# FASCICULE DE BREVET EUROPEEN

(11) **EP 2 074 986 B1**
(45) Date de publication et mention de la délivrance du brevet: **15.05.2013**
(21) Numéro de dépôt: 08171220.0
(22) Date de dépôt: 10.12.2008
(51) Int. Cl.: A61K 8/898, A61Q 5/06

(54) **Procédé de traitement des fibres capillaires au moyen de polysiloxane polyurée**
Haarbehandlungsmethode mittels Polysiloxane/Polyharnstoffe
Process for treating hair fibres using polysiloxane/polyurea

(30) Priorité: 13.12.2007 FR 0759812
(43) Date de publication de la demande: 01.07.2009
(73) Titulaire: L'Oréal, 75008 Paris (FR)
(72) Inventeur: Vic, Gabin, 45400, Semoy (FR); Brun, Gaëlle, 75015, Paris (FR); Gourlaouen, Luc, 92600, Asnieres (FR)
(74) Mandataire: Bourdeau, Françoise

(56) Documents cités:
- EP-A- 1 582 198
- WO-A-2005/087186
- FR-A- 2 743 297

## Description

L'invention concerne un procédé de traitement des fibres capillaires au moyen d'un polymère particulier.

Il existe de nombreux produits de coiffage permettant de mettre en forme les cheveux. Notamment les gels ou les mousses de coiffage permettent de structurer une chevelure sous forme de méchés ou de formes plus sophistiquées comme des boucles ou des crans si un outil chauffant approprié est utilisé. Cependant les mèches obtenues avec ces produits sont très rigides, manquent de brillance et de tenue dans le temps. C'est-à-dire qu'au cours de la journée la forme de ces mèches s'estompe rapidement.

Un autre problème important lié à l'utilisation des gels et des mousses avec un outil chauffant de type fer est le fait que la forme donnée à la mèche de cheveu est difficilement modifiable par la suite. Les propriétés de mise en forme du produit sont perdues lorsque'on réapplique de la chaleur. Il se forme aussi des dépôts sur les outils chauffant qui nécessitent un nettoyage réguliers des équipements..

Par ailleurs, d'autres procédés de traitement des cheveux utilisent une étape de chauffage des cheveux. Cependant, le chauffage des cheveux peut avoir un effet dégradant sur les fibres.

De nombreux procédés de traitement des cheveux utilisent des composés siliconés. Il est en effet connu que les composés siliconés sont des actifs cosmétiques qui améliorent les propriétés cosmétiques des cheveux. Ils ont un effet conditionneur sur les cheveux et apportent de la brillance.

Cependant, cet apport de brillance apporté par les silicones a tendance à s'estomper rapidement avec le temps.

Le but de la présente invention est de fournir un procédé de traitement des fibres capillaires qui permet de mettre facilement en forme les cheveux et de leur imprimer un relief souhaité. Cette mise en forme doit être thermoréversible c'est-à-dire qu'une nouvelle forme peut être donnée à la mèche de cheveu si elle est soumise à la chaleur à nouveau sans apport supplémentaire de formule. On souhaite aussi obtenir une très bonne brillance et un très bon toucher, et ceci durablement.

La demande de brevet EP 1 582 198, divulgue un procédé de traitement des fibres capillaires comprenant les étapes suivantes :
- application sur les fibres capillaires d'une composition comprenant au moins 5% en poids, par rapport au poids total de la composition, d'au moins une silicone choisie parmi les silicones arylées et les gommes de silicone, puis
- élévation de la température des fibres capillaires, au moyen d'un fer plat chauffant, à une température comprise entre 150 et 250°C.

Les résultats obtenus avec ce procédé ne permettent cependant pas d'obtenir les effets souhaités notamment en terme de mise en forme des cheveux.

La Demanderesse a trouvé qu'il était possible de remédier aux inconvénients de l'art antérieur et de remplir les objectifs précités.

Ainsi, l'invention a pour objet un procédé de traitement des fibres capillaires comprenant les étapes suivantes :
- application sur les fibres capillaires d'une composition cosmétique comprenant, dans un milieu cosmétiquement acceptable un ou plusieurs copolymères bloc polysiloxane polyurée, puis ou simultanément
- élévation de la température des fibres capillaires à une température comprise entre 50 et 280°C.
   L'application d'une telle composition sur le cheveu est très facile. Il se forme après traitement par la chaleur un méché homogène (groupe de cheveux liés). Le dépôt de polymère formé sur les cheveux est homogène, lisse et possède une excellente adhésion. Le toucher cosmétique des cheveux ainsi enrobés et notamment leur douceur est très bon. La brillance est également très bonne.
   Les propriétés obtenues sont durables, c'est-à-dire ne nécessitent pas de réapplication de produit pour durer dans le temps.
   Cette mise en forme est thermoréversible c'est-à-dire qu'une nouvelle forme peut être donnée à la mèche de cheveu si elle est soumise à nouveau à la chaleur sans apport supplémentaire de formule.

### COPOLYMERE BLOC POLYSILOXANE POLYUREE

Dans le cadre de l'invention on entend par copolymère bloc, un copolymère constitué d'au moins deux séquences distinctes de chacun des polymères constituant le copolymère dans le squelette du copolymère. Par exemple, le copolymère de l'invention contient au moins une séquence (ou bloc) de polysiloxane et au moins une séquence (bloc) de polyurée dans le squellette du copolymère.

Le copolymère de l'invention peut comprendre en plus du polysiloxane/polyurée d'autres blocs de motifs différents. On citera en particulier les terpolymères blocs polysiloxane/polyurée/polyuréthane.

Selon un mode de réalisation particulier, le copolymère contient une quantité en poids de polysiloxane supérieure à 5 %. Selon un mode de réalisation particulier, la quantité de polysiloxane est majoritaire dans le copolymère, de préférence supérieure à 90 % en poids par rapport au poids total du copolymère.

Selon une variante, le copolymère contient uniquement un ou plusieurs blocs siloxane et un ou plusieurs blocs polyurée.

Selon l'invention, le copolymère peut répondre à la formule générale (I) : 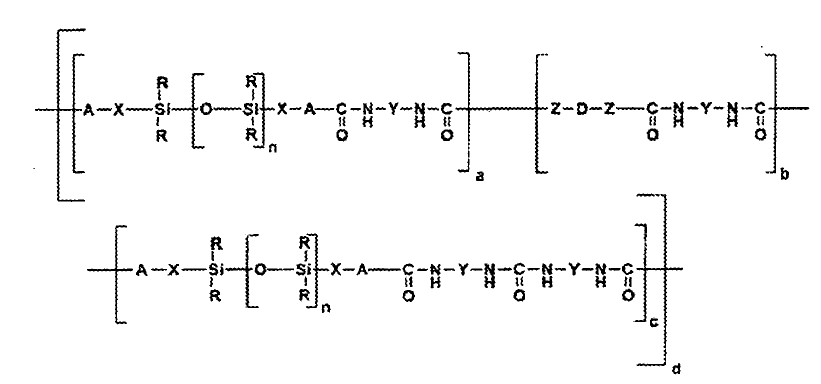 dans laquelle
R représente un radical hydrocarboné monovalent, le cas échéant substitué par le fluor ou le chlore, ayant 1 à 20 atomes de carbone,
X représente un radical alkylène ayant 1 à 20 atomes de carbone, dans lequel des unités méthylène non voisines peuvent être remplacées par des radicaux -O-,
A représente un atome d'oxygène ou un radical amino -NR'-,
Z représente un atome d'oxygène ou un radical amino -NR'-,
R' représente hydrogène ou un radical alkyle ayant 1 à 10 atomes de carbone,
Y représente un radical hydrocarboné bivalent, le cas échéant substitué par le fluor ou le chlore, ayant 1 à 20 atomes de carbone,
D représente un radical alkylène, le cas échéant substitué par fluor, chlore, alkyle en C₁-C₆ ou ester d'alkyle en C₁-C₆, ayant 1 à 700 atomes de carbone, dans lequel des unités méthylène non voisines peuvent être remplacées par des radicaux -O-, -COO-, - OCO- ou -OCOO-,
   - n: est un nombre allant de 1 à 4000,
   - a: est un nombre d'au moins 1,
   - b: est un nombre allant de 0 à 40,
   - c: est un nombre allant de 0 à 30, et
   - d: est un nombre supérieur à 0.
à la condition que A représente dans au moins un des motifs (a) un radical NH

De préférence, R représente un radical hydrocarboné monovalent avec 1 à 6 atomes de carbone par exemple méthyle, éthyle, vinyle et phényle. Selon un mode de réalisation particulier, R est un radical alkyle non substitué.

De préférence, X représente un radical alkylène avec 2 à 10 atomes de carbone. De préférence, le radical alkylène X n'est pas interrompu.

Selon un mode de réalisation particulier, le groupement A dans tous les motifs (b) et (c), lorsqu'ils sont présents, représente NH.

Selon un mode de réalisation particulièrement préféré, tous les groupements A représentent un radical NH.

De préférence, Z représente un atome d'oxygène ou un radical NH.

De préférence, Y représente un radical hydrocarboné comprenant de 3 à 13 atomes de carbone, qui est de préférence, non substitué. De préférence, Y représente un radical aralkylène, alkylène, linéaire ou cyclique.

De préférence, D représente un radical alkylène avec au moins 2, en particulier au moins 4 atomes de carbone et au maximum 12 atomes de carbone.

De préférence également, D représente un radical polyoxyalkylène, en particulier un radical polyoxyéthylène ou polyoxypropylène avec au moins 20, en particulier au moins 100 atomes de carbone et au maximum 800, en particulier au maximum 200 atomes de carbone.

De préférence, le radical D n'est pas substitué.

De préférence, n représente un nombre d'au moins 3, en particulier au moins 25 et de préférence, au maximum 800, en particulier au maximum 400, de manière particulièrement préférée, au maximum 250.

De préférence, a représente un nombre de plus de 50.

Lorsque b est différent de 0, b représente de préférence, un nombre d'au maximum 50, en particulier au maximum 25.

De préférence, c représente un nombre d'au maximum 10, en particulier au maximum 5.

Les copolymères de l'invention peuvent être obtenus selon les procédés de polymérisation décrits dans la demande de brevet US 2004/0254325 ou la demande WO 03/014194.

Le copolymère peut ainsi être obtenu par un procédé en deux étapes, tel que :
- dans la première étape, on fait réagir un silazane de formules générales (2) ou (2'): 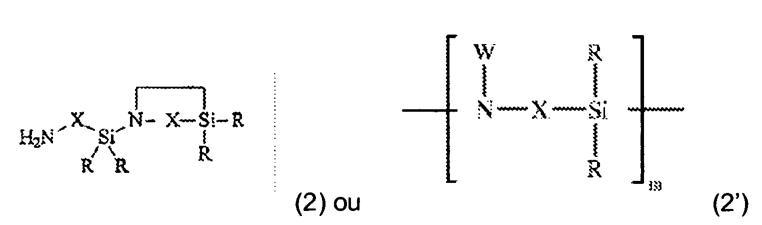 W représentant un atome d'hydrogène, un radical hydrocarboné substitué ou non, comprenant de préférence de 1 à 20 atomes de carbone ou un radical R₂Si-X-NH₂;

   (HO)(R₂SiO)ₙ₋₁[H] (3)

   avec un composé organique du silicium de la formule générale (3) :
   pour obtenir un aminoalkylpolydiorganosiloxane de la formule générale (4)

   H₂N-X-[SiR₂O]ₙSiR₂-X NH₂ (4)
- dans une deuxième étape, l'aminoalkyl-polydiorganosiloxane de la formule générale (4) est polymérisé avec un diisocyanate de la formule générale (5) :

   OCN-Y-NCO (5)

De manière générale, dans la première étape, on met en oeuvre les silazanes de formule générale (2) ou (2') et les réactifs contenant des groupes silanol en rapports équimolaires.

Pour la préparation des silicones à terminaison bisaminoalkyle très purs, de la formule générale (4), on utilise de préférence, un petit excès du composé silazane de la formule générale (2) ou (2'), qui peut être ensuite éliminé, dans une étape de procédé supplémentaire simple, comme par exemple, l'addition de faibles quantités d'eau.

Si b est au moins 1, on peut mettre en oeuvre au cours de la deuxième étape, jusqu'à 95% en poids, sur base de tous les composants mis en oeuvre, d'agents d'allongement de chaîne, qui sont choisis parmi les diamines, les composés hydroxy bloqués par un isocyanate, les composés dihydroxy ou leurs mélanges.

De préférence, les agents d'allongement de chaîne présentent la formule générale (6) :

HZ-D-ZH (6)

où D et Z présentent les significations précédentes. Si Z a la signification O, l'agent d'allongement de chaîne de la formule générale (6) peut également être mis à réagir avant la réaction dans la deuxième étape, avec le diisocyanate de la formule générale (5). Le cas échéant, on peut mettre en oeuvre de l'eau comme agent d'allongement de chaîne.

Des exemples de diisocyanates de la formule générale (5) à utiliser, sont des composés aliphatiques comme l'isophoronediisocyanate, l'hexaméthylène-1,6-diisocyanate, le tétraméthylène-1,4-diisocyanate et le méthylènedicyclohexyl-4,4'-diisocyanate ou des composés aromatiques comme le méthylènediphényl-4,4'-diisocyanate, le 2,4-toluènediiso-cyanate, le 2,5-toluènediisocyanate, le 2,6-toluènediiso-cyanate, le m-phénylènediisocyanate, le p-phénylène-diisocyanate, le m-xylènediisocyanate, le tétraméthyl-m-xylènediisocyanate ou des mélanges de ces isocyanates. Un exemple de composé disponible dans le commerce est un diisocyanate de la série DESMODUR® (H, I, M, T, W) de Bayer AG, Allemagne. On préfère les diisocyanates aliphatiques, dans lesquels Y est un radical alkylène, parce que ceux-ci conduisent à des matériaux, qui présentent des stabilités aux U.V. améliorées,.

Les alkylènes à terminaison α,ω-OH de la formule générale (6) sont de préférence, des polyalkylènes ou des polyoxyalkylènes. Ceux-ci sont de préférence, essentiellement exempts de contaminations de polyoxyalkylènes mono-, trifonctionnels ou de fonctionnalité supérieure. On peut mettre en oeuvre ici, des polyétherpolyols, polytétraméthylènediols, polyesterpolyols, polycaprolactone-diols, mais également des polyalkylènes à terminaison α ,ω-OH à base de poly(acétate de vinyle), de copolymères poly(acétate de vinyle)-éthylène, de copolymères poly(chlorure de vinyle), de polyisobutyldiols. De préférence, on utilise des polyoxyalkyles, de manière particulièrement préférée, des polypropylèneglycols. De tels composés sont disponibles dans le commerce comme matériaux de base entre autres, pour des mousses polyuréthanne et pour des utilisations comme revêtement, avec des masses moléculaires Mn de jusqu'à 10 000. Des exemples sont les polyétherpolyols et polyesterpolyols BAYCOLL® de BAYER AG, Allemagne ou les polyétherpolyols Acclaim® de Lyondell Inc., USA. On peut mettre en oeuvre également, des monomères α,ω-alkylènediols, comme l'éthylèneglycol, le propanediol, le butanediol ou l'hexanediol. D'autre part, par composés dihydroxylés dans le sens de l'invention, on entend également les bishydroxyalkyl-silicones, comme elles sont fournies par exemple par la société Goldschmidt sous les noms Tegomer H-Si 2111, 2311 et 2711.

La préparation des copolymères décrits ci-dessus de la formule générale (I) peut se faire en solution, mais également sous forme solide, de manière continue ou discontinue.

Si la quantité de segments uréthane ou urée est grande, on choisit un solvant ayant un paramètre élevé de solubilité, comme par exemple le diméthylacétamide. On peut également utiliser le THF. Selon un mode de réalisation particulier, la synthèse du copolymère est mise en oeuvre sans solvant.

La synthèse est de préférence réalisée en l'absence d'humidité et sous gaz protecteur, usuellement l'azote ou l'argon.

La réaction est réalisée de préférence en présence d'un catalyseur. Les catalyseurs appropriés pour la préparation sont des composés dialkylétain, comme par exemple le dilaurate de dibutyl-étain, le diacétate de dibutyl-étain, ou des amines tertiaires comme par exemple la N,N-diméthylcyclohexanamine, le 2-diméthylaminoéthanol, la 4-diméthylaminopyridine.

Selon un mode de réalisation particulier, le copolymère utile dans la présente invention ne contient pas de polyuréthane.

Selon un autre mode de réalisation, le copolymère est un copolymère polysiloxane/polyurée non ionique, c'est-à-dire qu'il ne contient pas de groupement ionisé ou ionisable.

A titre d'exemple de copolymère, on peut citer le copolymère diméthylpolysiloxane/urée, de nom INCI polyureadimethicone.

Un tel polymère peut être obtenu notamment par copolymérisation d'un alpha,oméga-aminosilicone avec un di-isocyanate. Des polymères répondant à ces caractéristiques sont par exemple les produits commercialisés sous la référence Wacker-Belsil ® UD 60 Wacker-Belsil ® UD 80, Wacker-Belsil ® UD 140 et Wacker-Belsil ® UD 200 par la société Wacker.

Selon un mode de réalisation particulier, le copolymère polyurée/polysiloxane est non ionique.

De préférence le ou les copolymères bloc polysiloxane polyurée représentent de 0.05 à 20%, mieux de 0.1 à 15% et encore plus mieux de 0.5 à 10% du poids de la composition appliquée..

### CHARGE

La composition peut contenir des charges qui sont généralement des composés substantiellement non colorés solides à température ambiante et pression atmosphérique, et insoluble dans la composition, même lorsque ces ingrédients sont portés à une température supérieure de la température ambiante.

Les charges peuvent être minérales ou organiques. Les charges peuvent être des particules de toute forme, notamment plaquettaires, sphériques ou oblongues, quelle que soit leur forme cristallographique (par exemple feuillet, cubique, hexagonale, orthorhombique). De plus ces particules peuvent être pleines, creuses ou poreuses, enrobées ou non.

Parmi les charges utilisables dans les compositions selon l'invention, on peut notamment citer des charges minérales telles que le talc ; le mica naturel ou synthétique ; la silice ; le kaolin ; le nitrure de bore, le carbonate de calcium précipité; le carbonate et l'hydro-carbonate de magnésium ; l'hydroxyapatite.

Ces charges minérales peuvent se présenter sous la forme de particules sphériques avec par exemple les microsphères de silice creuses telles que les 'SILICA BEADS SB 700/HA®' ou 'SILICA BEADS SB 700®' de la société MAPRECOS, les 'SUNSPHERES H-33®' et les 'SUNSPHERES H-51®' de la société ASAHI GLASS.

Avantageusement, la ou les particules inorganiques présentent une taille primaire moyenne en nombre comprise entre 0,1 et 30 µm, de préférence comprise entre 0,2 et 20 µ m, et encore plus préférentiellement comprise entre 0,5 et 15 µ m. Au sens de la présente invention, on entend par « taille primaire de particule », la dimension maximale qu'il est possible de mesurer entre deux points diamétralement opposés d'une particule individuelle. La taille des particules organiques peut être déterminée par microscopie électronique à transmission ou à partir de la mesure de la surface spécifique par la méthode BET ou à partir d'une granulométrie laser.

De préférence les charges inorganiques utilisées selon l'invention sont la silice, le talc, le nitrure de bore.

Parmi les charges utilisables dans les compositions selon l'invention, on peut notamment citer des charges organiques. Par charge organique, on entend une particule polymérique qui peut être issue de la polymérisation d'un ou de plusieurs monomères. Les polymères constituant ces particules organiques peuvent être réticulés ou non. Les monomères utilisés peuvent être en particulier des esters d'acide méthacrylique ou acrylique, tels que l'acrylate et méthacrylate de méthyle, le chlorure de vinylidène, l'acrylonitrile, le styrène et ses dérivés.

Avantageusement, la ou les particules organiques présentent une taille primaire moyenne en nombre comprise entre 1 et 30 µ m, de préférence comprise entre 1 et 20 µ m, et encore plus préférentiellement comprise entre 1 et 15 µ m.

La ou les particules organiques utilisées dans la composition cosmétique selon l'invention peuvent être choisies parmi les poudres de polyamide, les poudres de polymères acryliques, notamment de polyméthacrylate de méthyle, les poudres de copolymères acryliques, notamment de polyméthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, de polyacrylate/alkylacrylate, les poudres de polystyrène, les poudres de polyéthylène, notamment de polyéthylène/acide acrylique, les microbilles de résine de silicone.

A titre représentatif et non limitatif, on peut particulièrement citer en tant que particules organiques selon l'invention :
- les poudres de polyamides (Nylon ®), par exemples celles commercialisées sous les dénominations « ORGASOL ® 4000 » et « ORGASOL ® 2002 UD NAT COS 204 » par la société ATOCHEM,
- les poudres de polymères acryliques, notamment de polyméthacrylate de méthyle, comme par exemples celles commercialisées sous la dénomination « COVABEAD ® LH85 » « COVABEAD ® PMMA »par la société WACKHERR ou celles commercialisées sous la dénomination « MICROPEARL ® MHB » commercialisée par la société MATSUMOTO,
- les poudres de copolymères acryliques, notamment de polyméthacrylate de méthyle/diméthacrylate d'éthylène glycol, comme celles commercialisées sous la dénomination de « DOW CORNING 5640 MICROSPONGE ® SKIN OIL ADSORBER » par la société DOW CORNING, ou celles commercialisés sous la dénomination « GANZPEARL ® GMP-0820 » par la société GANZ CHEMICAL, de polyméthacrylate d'allyle/diméthacrylate d'éthylène glycol, comme celles commercialisées sous la dénomination « POLYPORE ® L200 » ou « POLYPORE ® E200 » commercialisés par la société AMCOL, de copolymère diméthacrylate d'éthylène glycol / méthacrylate de lauryle, comme celles commercialisées par « POLYTRAP ® 6603 » par la société DOW CORNING, de polyacrylate/éthylhexylacrylate comme celles commercialisées sous la dénomination « TECHPOLYMER ® ACX 806C » par la société SEKISUI,
- les poudres de polystyrène / dinvinylbenzène comme celles commercialisées sous la dénomination « TECHPOLYMER ® SBX8 » par la société SEKISUI,
- les poudres de polyéthylène, notamment de polyéthylène/acide acrylique commercialisées sous la dénomination « FLOBEADS ® » par la société SUMITOMO,
- les microbilles de résine de silicone, comme celles commercialisées sous les dénominations « TOSPEARL ® » par la société TOSHIBA SILICONE, en particulier les « TOSPEARL ® 240A » et « TOSPEARL ® 120A ».
- les microsphères de polymères acryliques telles que celles en copolymère d'acrylate réticulé 'POLYTRAP 6603 ADSORBER®' de la société RP SCHERRER
- les poudres de polyuréthane telle que la poudre de copolymère de diisocyanate d'hexaméthylène et de triméthylol hexyl lactone vendue sous la dénomination « PLASTIC POWDER D-400® » par la société Toshiki
- les microcapsules de polymères ou de copolymères d'acrylate ou de méthacrylate de méthyle, ou encore de copolymères de chlorure de vinylidène et d'acrylonitrile, comme l' « EXPANCEL® » de la société EXPANCEL
- les poudres d'organopolysiloxane réticulés élastomères telles que celles vendues sous la dénomination « TREFIL POWDER E-506C » par la société DOW CORNING
- les poudres polyfluorées notamment de polytétrafluoroéthylène, par exemple celle commercialisée sous la dénomination "MP 1400" par la Société DUPONT DE NEMOURS,

De préférence, les particules organiques utilisées dans la composition conforme à l'invention sont choisies parmi les poudres de polyamide et les poudres de polyméthylméthacrylate.

Les espèces colorées ou colorantes ainsi que les charges peuvent être présents dans la composition en quantité comprise entre 0,001 et 20 % en poids environ du poids total de la composition, notamment entre 0,1 et 10 %.

La composition de l'invention peut aussi contenir une espèce colorée ou colorante telle que des pigments colorés, des colorants directs hydrophiles ou hydrophobes ou des précurseurs de colorants. Les pigments colorés et les colorants peuvent être fluorescent ou non.

Par espèce coloré, on entend dans le cadre de l'invention un composé coloré à l'état sec ou en solution, c'est-à-dire absorbant entre 250 et 750 nm et/ou réémettant par excitation une lumière visible. Par espèce colorante, on entend une espèce non colorée générant un ou plusieurs composés colorés par interaction avec un agent réactif, tel qu'un agent oxydant.

A titre de colorant hydrophile, on peut citer les colorants présentant une hydrophilicité définie par la valeur de logP inférieur ou égale à 2. Dans le cadre de l'invention, la valeur du logP représente de façon classique le coefficient de partage du colorant entre l'octanol et l'eau. La valeur du logP peut être calculée selon la méthode décrite dans l'article de Meylan et Howard « Atom / Fragment contribution method for estimating octanol-water partition coefficient », J. Pharm. Sci., 1995, 84, p83-92. Cette valeur peut aussi être calculée à partir de nombreux logiciels disponibles sur le marché qui détermine la valeur de logP en fonction de la structure d'une molécule.

A titre d'exemple, on peut citer le logiciel Epiwin de l'agence de l'environnement des Etats-Unis.

De façon avantageuse, LogP des colorants hydrohiles utiles dans la composition de l'invention est strictement inférieur à 2.

Parmi ces colorants hydrophiles, on peut citer les colorants directs nitrés benzéniques neutres ; acides ou cationiques ; les colorants directs azoïques neutres acides ou cationiques ; les colorants directs quinoniques et en particulier anthraquinoniques neutres, acides ou cationiques ; les colorants directs aziniques ; les colorants directs triarylméthaniques ; les colorants directs indoaminiques et les colorants directs naturels.

Ainsi, les colorants directs peuvent être choisis en fonction de la valeur LogP.

| **STRUCTURE** | **NOM** | **log P** |
|---|---|---|
| 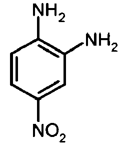 | 4-nitro-o-phénylène-diamine | 0.88 |
| 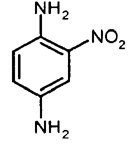 | 2-nitro-p-phénylène-diamine | 0.53 |
| 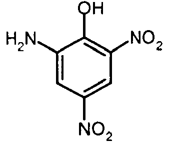 | acide picramique | 0.93 |
| 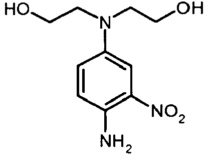 | HC Red 13 | 0.66 |
| 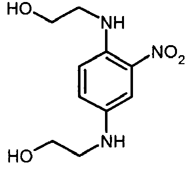 | N,N'-bis-(2-hydroxyéthyl)-2-nitro-p-phénylène-diamine | -0.44 |
| 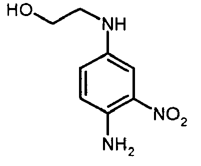 | HC Red 7 | 0.13 |
| 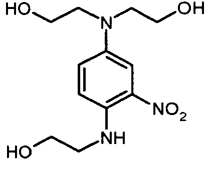 | HC Blue 2 | -0.32 |
| 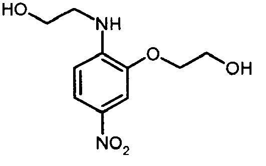 | HC Yellow 4 | 0.56 |
| 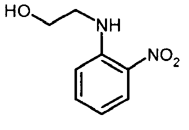 | HC Yellow 2 | 1.05 |
| 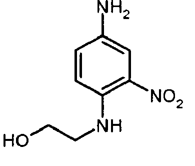 | HC Red 3 | -0.42 |
| 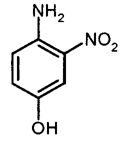 | 4-amino-3-nitrophénol | 1.19 |
| 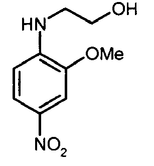 | 1-hydroxyéthyl-amino-5-nitroanisole | 1.13 |
| 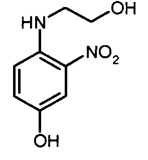 | 3-nitro-p-hydroxyéthylamino phénol | 0.21 |
| 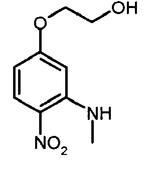 | 3-méthylamino-4-nitrophénoxyéthanol | 1.13 |
| 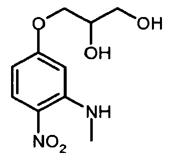 | 2-nitro-5-glycéryl méthylaniline | 0.89 |
| 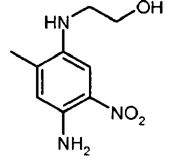 | HC Violet 1 | 0.67 |
| 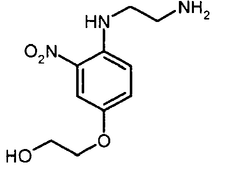 | HC Orange 2 | 0.15 |
| 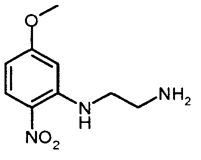 | HC Yellow 9 | 1.12 |
| 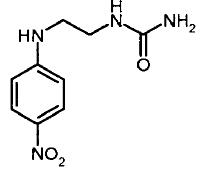 | 4-nitrophényl aminoéthylurée | 0.59 |
| 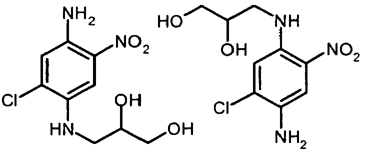 | HC Red 10 et HC Red 11 | 0.13 |
| 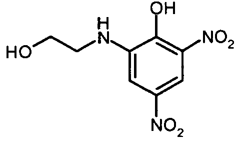 | Acide 2-hydroxyéthyl picramique | 0.38 |
| 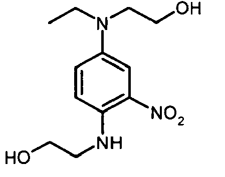 | HC Blue 12 | 1.15 |
| 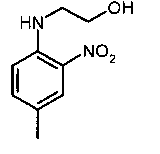 | 3 nitro 4 N beta hydroxyéthyl aminotoluène | 1.59 |
| 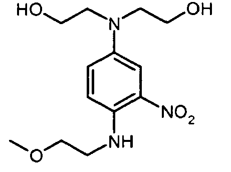 | 2 N béta méthoxyéthylamino 5 N,Nbis (hydroxyéthyl) amino nitrobenzène | 0.38 |
| 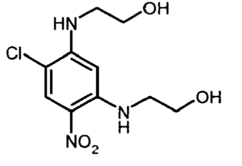 | HC Yellow 10 | 0.20 |
| 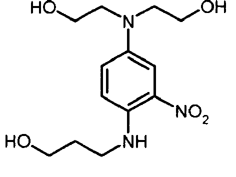 | HC Violet 2 | 0.17 |
| 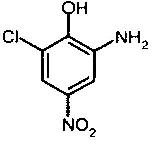 | 2-amino-6-chloro-4-nitrophénol | 1.53 |
| 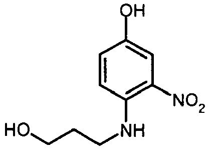 | 4-hydroxypropyl-amino-3-nitrophénol | 0.70 |
| 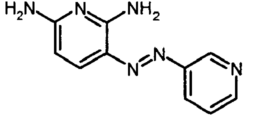 | 2,6-diamino-3-((pyridine-3-yl)-azo)pyridine | 1.58 |
| 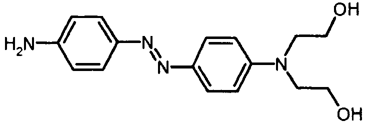 | Disperse Black 9 | 1.83 |
| 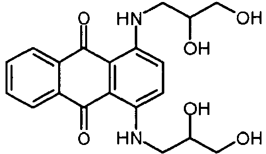 | HC Blue 14 | 0.62 |

La composition de l'invention peut aussi contenir des colorants hydrophobes. Dans le cadre de l'invention, l'hydrophobicité est définie par la valeur de logP qui doit être supérieure à 2.

A titre d'exemple, on peut citer :

| **Colorant** | **Structure chimique** | **logP** |
|---|---|---|
| Solvent Black 3 | 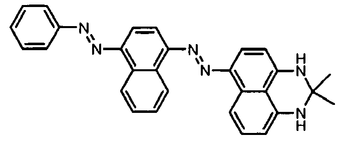 | 8.81 |
| Solvent Blue 104 | 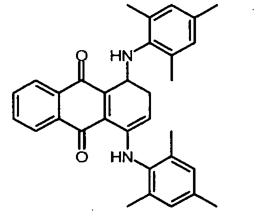 | 8.26 |
| Disperse Blue 134 | 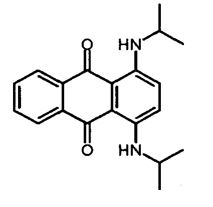 | 6.07 |
| Solvent Blue 14 | 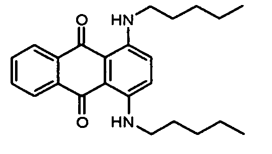 | 8.18 |
| Disperse Blue 14 | 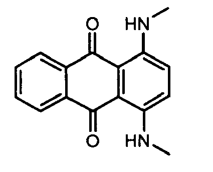 | 4.25 |
| Solvent Red 2 | 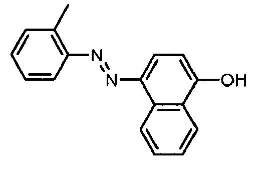 | 5.35 |
| Solvent Brown 5 | 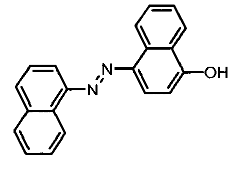 | 5.98 |
| Solvent Green 5 | 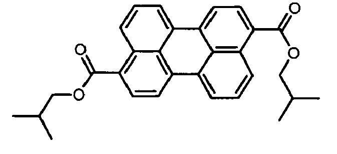 | 8.55 |
| Solvent Orange 2 | 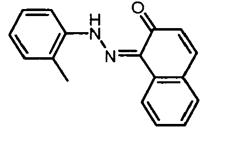 | 3.86 |
| Solvent Orange 1 | 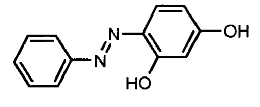 | 3.85 |
| Disperse Orange 24 | 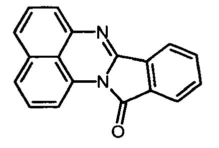 | 3.21 |
| Solvent Orange 63 | 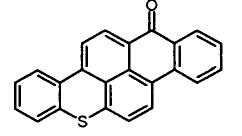 | 7.02 |
| Solvent Red 49 | 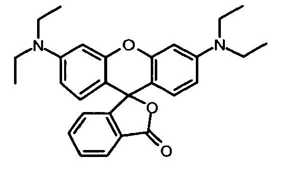 | 6.63 |
| Solvent Red 1 | 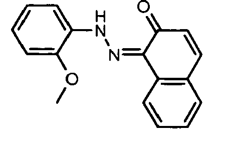 | 3.39 |
| Solvent Red 26 | 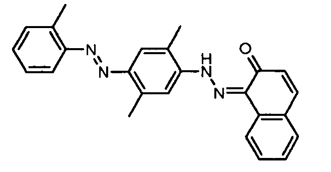 | 7.07 |
| Solvent Red 27 | 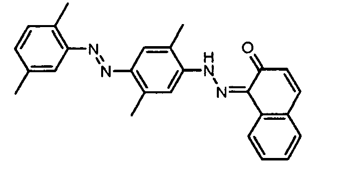 | 7.62 |
| Solvent Red 18 | 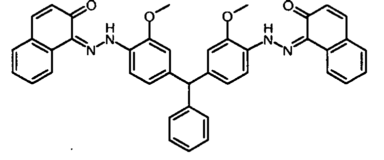 | 8.16 |
| Solvent Red 23 | 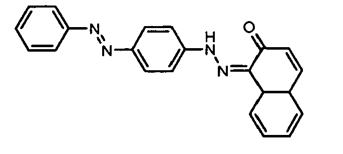 | 5.58 |
| Solvent Red 4 | 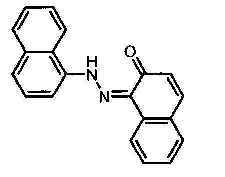 | 4.48 |
| Solvent Orange 7 | 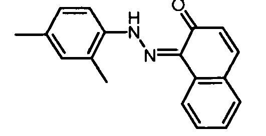 | 4.40 |
| Disperse Blue 72 | 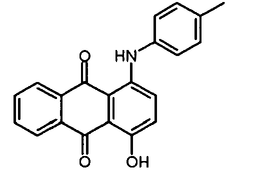 | 6.24 |
| Disperse Violet 26 | 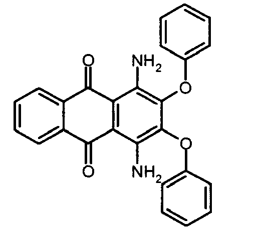 | 5.19 |
| Disperse Yellow 16 | 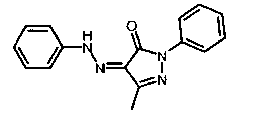 | 3.89 |
| Disperse Yellow 82 | 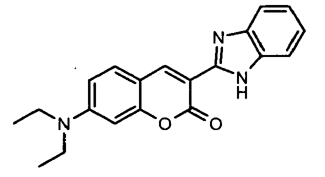 | 3.68 |
| Disperse Yellow 54 | 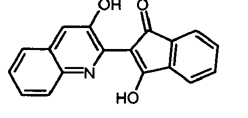 | 4.76 |
| Solvent Yellow 29 | 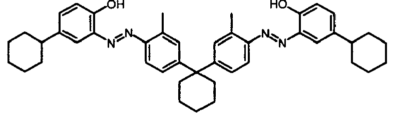 | 17.37 |
| Solvent Yellow 163 | 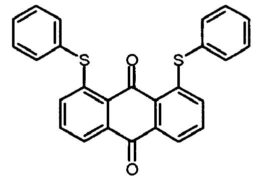 | 7.94 |
| Solvent Yellow 3 | 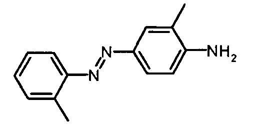 | 4.29 |
| Solvent Yellow 56 | 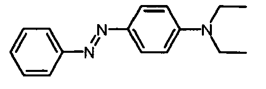 | 5.27 |
| Solvent Yellow 18 | 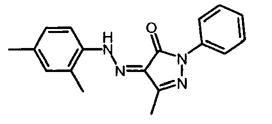 | 4.98 |
| Solvent Yellow 98 | 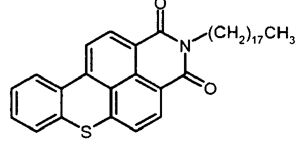 | 4.5 |
| Solvent Yellow 12 | 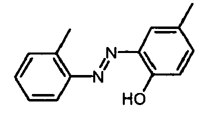 | 5.43 |
| Solvent Yellow 14 | 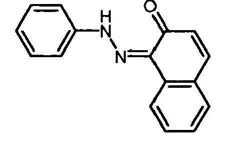 | 3.31 |
| Disperse Red 13 | 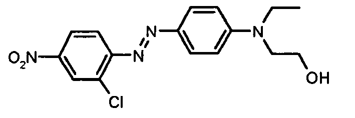 | 5.22 |
| Disperse Green 9 | 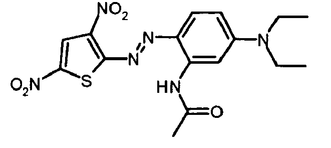 | 4.23 |
| Disperse Blue 148 | 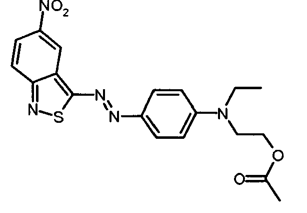 | 4.81 |
| Disperse Violet 63 | 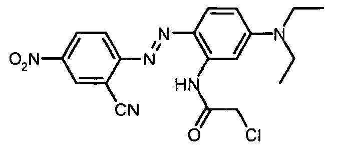 | 5.30 |
| Disperse Blue 60 | 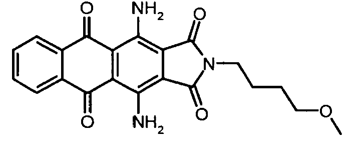 | 3.38 |
| Solvent Orange 15 | 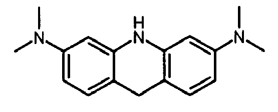 | 3.90 |

De préférence les colorants hydrophobes ont un logP supérieur à 4 et encore plus préférentiellement supérieur à 6.

L'utilisation de colorants fluorescents peut permettre d'obtenir sur cheveux foncés des colorations plus visibles qu'avec des colorants directs hydrophiles ou hydrophobes traditionnels. De plus ces colorants fluorescents lorsqu'ils sont appliqués sur des cheveux foncés peuvent également permettre de les éclaircir sans les dégrader. Cette technique décrite notamment dans le document FR 2830189 permet de respecter la qualité de la fibre kératinique lors du traitement mais les colorants fluorescents employés ne présente pas une bonne résistance aux shampoings. La présence du copolymère PDMS-polyurée permet d'améliorer cette rémanence aux shampooings.

On entend par ailleurs par composé fluorescent des colorants fluorescents et des azurants optiques. Ces composés fluorescents doivent eux aussi être solubles dans le milieu de la composition.

Les colorants fluorescents sont des composés fluorescents qui absorbent le rayonnement visible généralement entre 400 et 800 nm et qui sont capables de réémettre de la lumière dans le domaine du visible à une longueur d'onde supérieure. Par définition, ces colorants sont des espèces colorées puisqu'elles absorbent de la lumière visible.

Selon un mode de réalisation particulier, les colorants fluorescents utiles dans le cadre de l'invention réémettent de la lumière fluorescente orangée. Ils présentent notamment une longueur d'onde maximum de ré-émission comprise entre comprise entre 500 et 700 nm.

A titre d'exemple de colorants fluorescents, on peut citer les composés connus de la technique, décrits par exemple dans les ouvrages suivants : Ullmann's Encyclopedia of Industrial Chemistry Release 2004, 7th edition, chapitre « Fluorescent Dyes ».

Les azurants optiques utiles dans la présente invention aussi connus sous le nom de "brighteners", ou "fluorescent brighteners", ou "fluorescent brightening agents" ou "FWA", ou "fluorescent whitening agents", ou "whiteners", ou encore "fluorescent whiteners" en terminologie anglo-saxone, sont des composés transparents incolores car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; en général dans le bleu et/ou le vert, c'est-à-dire dans des longueurs d'onde allant de 400 à 550 nanomètres.

Les azurants optiques sont connus de la technique. Ils sont par exemple décrits dans Ullmann's Encyclopedia of Industrial Chemistry (2002) "Optical Brighteners" et Kirk-Othmer Encyclopedia of Chemical Technology (1995); "Fluorescent Whitening Agents".

Les colorants fluorescents qui peuvent être utilisés dans le cadre de l'invention sont des composés connus de la technique. Ils sont par exemple décrits dans le document FR 2 830 189. A titre d'exemples de colorants fluorescents, on peut notamment citer :
- le Photosensitizing Dye NK-557 commercialisé par la société UBICHEM qui présente la structure suivante : 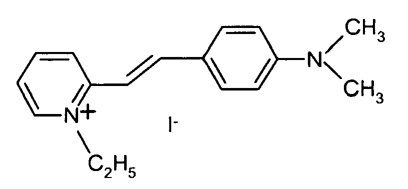 iodure de 2-[2-(4-diméthylamino)phényl éthényl]-1 éthyl-pyridinium ;
- le Jaune Brilliant B6GL commercialisé par la société SANDOZ et de structure suivante : 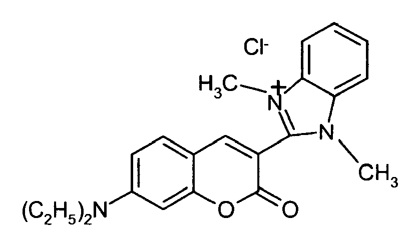
- le Basic Yellow 2, ou Auraminoe O commercialisé par les sociétés PROLABO, ALDRICH ou CARLO ERBA et de structure suivante : 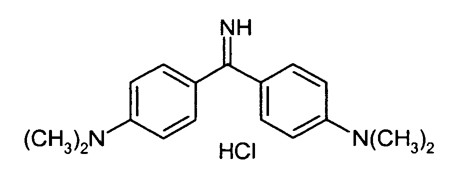 monochlorhydrate de 4,4'-(imidocarbonyl)bis(N,N-diméthylaniline)

A titre d'azurants optiques et de colorants fluorescents utiles dans la présente invention on peut aussi citer :
- Les naphthalimides, par exemple le composé suivant 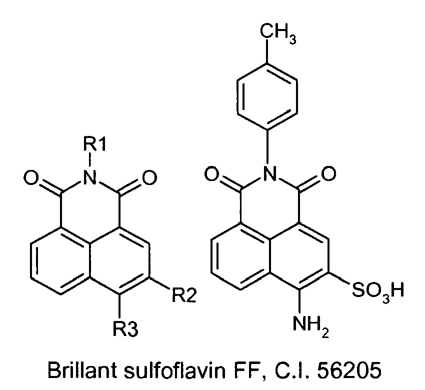 Dans laquelle R₁, R₂, R₃, indépendamment les uns des autres représentent un atome d'hydrogène ; un atome d'halogène ; un groupement aryle en C₆-C₃₀ ; un groupement hydroxy ; un groupement cyano ; un groupement nitro ; un groupement sulfo; un groupement amino; un groupement acylamino; un groupement dialkyl(C₁-C₆)amino; un groupement dihydroxyalkyl(C₁-C₆)amino; un groupement alkyl(C₁-C₆)hydroxyalkyl(C₁-C₆)amlno ; un groupement alcoxy(C₁-C₆); un groupement alcoxy(C₁-C₆)carbonyle; un groupement carboxyalcoxy en C₁-C₆; un groupement pipéridinosulfonyle ; un groupement pyrrolidino ; un groupement alkyle(C₁-C₆)halogénoalkyle(C₁-C₆)amino; un groupement benzoylalkyle(C₁-C₆); un groupement vinyle ; un groupement formyle; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène,ce radical alkyle pouvant être interrompu par un hétéroatome, tel que N, S ou O ; les substituants R₁, R₂, R₃ peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄).
- Les dérivés de coumarines tels que les composés correspondants aux formules suivantes 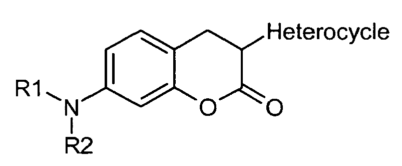 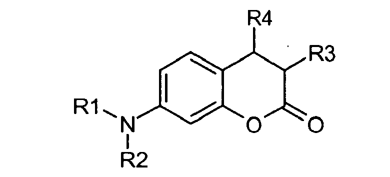
dans laquelle l'hétérocycle est choisi parmi les furane, tiophène, 2H-pyrrole, 2-pyrroline, pyrrolidine, 1,3-dioxolane, oxazole, thiazole, Imidazole, 2-imidazoline, Imidazoline, pyrazole, 2-pyrazoline, pyrazolidine, isoxazole, isothiazole, 1,2,3-oxadiazole, 1,2,3-triazole, 1,3,4-thiadiazole, 2H-pyran, 4H-pyran, pyridine, piperidine, 1,4-dioxane, morpholine, 1,4-dithiane, thiomorpholine, pyridazine, pyrimidine, pyrazine, piperazine, 1,3,5-Triazine, 1,3,5-trithiane, indolizine, indole, isoindole, 3H-indole, indoline, benzo[b]furan, benzo[b]thiophene, 1H-indazole, benzimidazole, benzothiazole, purine, 4H-quinolizine, quinoline, isoquinoline, cinnoline, phtalazine, quinazoline, quinoxaline, 1,8-naphtyridine, pteridine, quinuclidine, carbazole, acridine, phenazine, phenothiazine, phenoxazine, indene, naphtalene, azulene, fluorene, anthracene, norbornane, adamantane, et

R₁, R₂, R₃, R₄ indépendamment les uns des autres représentent un atome d'hydrogène; un atome d'halogène; un groupement aryle en C⁶-C₃₀; un groupement hydroxy ; un groupement cyano ; un groupement nitro ; un groupement sulfo; un groupement amino; un groupement acylamino; un groupement dialkyl(C₁-C₆)amino; un groupement dihydroxyalkyl(C₁-C₆)amino; un groupement alkyl(C₁-C₆)hydroxyalkyl(C₁-C₆)amlno; un groupement alcoxy(C₁-C₆); un groupement alcoxy(C₁-C₆)carbonyle; un groupement carboxyalcoxy en C₁-C₆; un groupement pipéridinosulfonyle ; un groupement pyrrolidino ; un groupement alkyle(C₁-C₆)halogénoalkyle(C₁-C₆)amino; un groupement benzoylalkyle(C₁-C₆); un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène, ce radical alkyle pouvant être interrompu par un hétéroatome, tel qu'un atome d'azote, de soufre ou d'oxygène ; les substituants R₁, R₂, R₃ et R₄ peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄);
deux des substituants R₃ , R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ce cycle étant condensés ou non,ce cycle et l'éventuel cycle condensé étant substitués ou non par au moins un groupement choisi parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄).

. A titre d'exemple, on peut citer 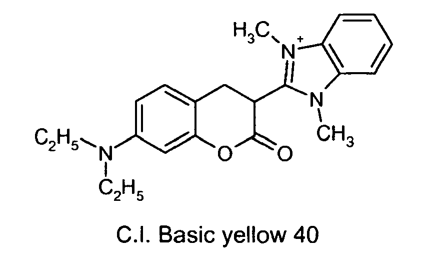
- Les dérivés de xanthènes tels que : 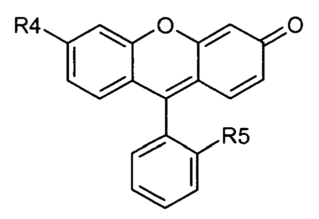 dans laquelle R₄ et R₅ indépendamment les uns des autres représentent un atome d'hydrogène ; un atome d'halogène ; un groupement aryle en C₆-C₃₀; un groupement hydroxy ; un groupement cyano ; un groupement nitro ; un groupement sulfo; un groupement amino; un groupement acylamino; un groupement dialkyl(C₁-C₆)amino; un groupement dihydroxyalkyl(C₁-C₆)amino; un groupement alkyl(C₁-C₆)hydroxyalkyl(C₁-C₆)amino; un groupement alcoxy(C₁-C₆); un groupement alcoxy(C₁-C₆)carbonyle; un groupement carboxyalcoxy en C₁-C₆; un groupement pipéridinosulfonyle ; un groupement pyrrolidino ; un groupement alkyle(C₁-C₆)halogénoalkyle(C₁-C₆)amino; un groupement benzoylalkyle(C₁-C₆); un groupement vinyle ; un groupement formyle; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène,ce radical alkyle pouvant être interrompu par un hétéroatome, tel que l'azote, le soufre et l'oxygène.
- Les rhodamines telles que 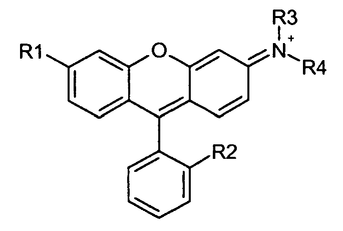
dans laquelle R₁, R₂, R₃ et R₄ sont tels que définis ci-dessus. A titre d'exemple, on peut citer 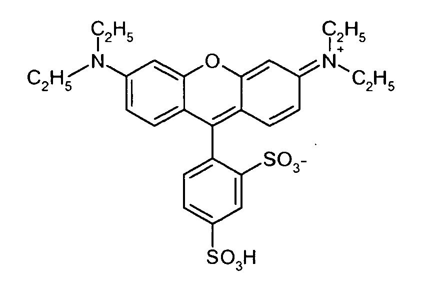 Sulforhodamine B C.I. 45100 Acid Red 52
- Les derives de thioxanthènes tels que 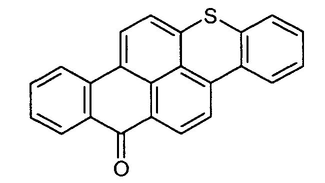 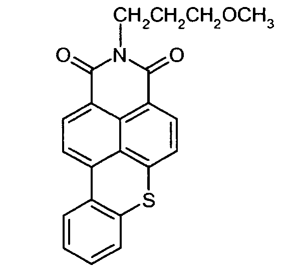 Samaron, Brilliant yellow H6GL, C.I. 56235 Disperse yellow 105
- Les dérivés de naphtolactames tels que : 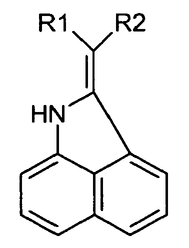 avec R₁ et R₂ définis comme précédemment
On peut citer par exemple le composé suivant 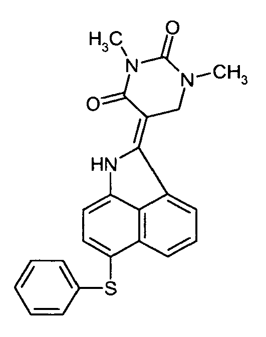 Disperse dye 28
• Les dérivés AZALACTONES : 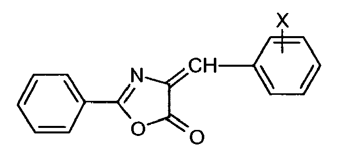 X ayant la même définition que R1 décrit précédemment.
   On peut citer par exemple le composé suivant 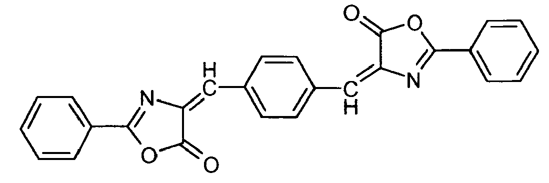
• Les dérivés méthiniques tels que 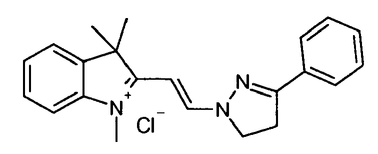 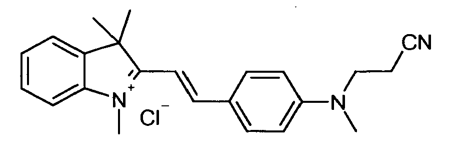
• Les dérivés oxazines et thiazines tels que 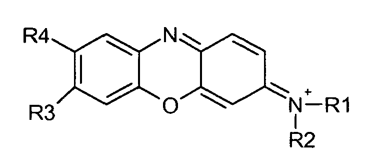 dans lesquelles R₁, R₂, R₃ et R₄ sont tels que définis ci-dessus. A titre d'exemple, on peut citer 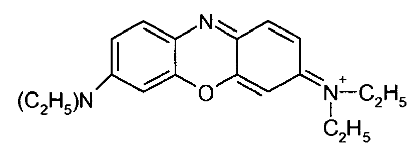 Basic Blue 3, C.I. 51004
• Les dérives de 1,4-DISTYRYLBENZENES de formule : 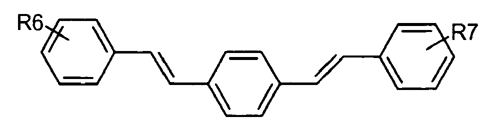 dans laquelle R6 et R7 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo; un groupement amino; un groupement acylamino; un groupement dialkyl(C1-C6)amino ; un groupement dihydroxyalkyl(C1-C6)amino ; un groupement alkyl(C1-C6)hydroxyalkyl(C1-C6)amino; un groupement alcoxy(C1-C6); un groupement alcoxy(C₁-C₆)carbonyle; un groupement carboxyalcoxy en C1-C6 ; un groupement pipéridinosulfonyle ; un groupement pyrrolidino ; un groupement alkyle(C1-C6)halogénoalkyle(C1-C6)amino; un groupement benzoylalkyle(C1-C6); un groupement vinyle ; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome, tel que N, S ou O.
• Les dérivés 4,4'-DISTYRYLBIPHENYLES de formule : 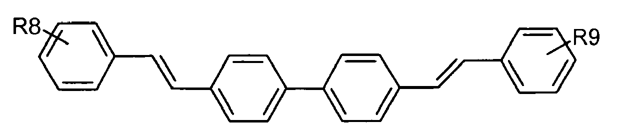 dans laquelle R8 et R9 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo; un groupement amino; un groupement acylamino; un groupement dialkyl(C1-C6)amino; un groupement dihydroxyalkyl(C1-C6)amino; un groupement alkyl(C1-C6)hydroxyalkyl(C1-C6)amino; un groupement alcoxy(C1-C6); un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6); un groupement vinyle ; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
• Les dérivés TRIAZINYLAMINOSTILBENES de formule : 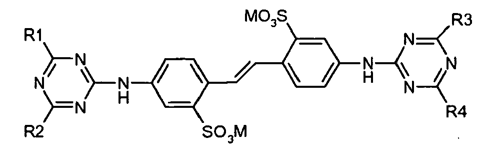 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6); un groupement alcoxy(C1-C6)carbonyle ; un groupement carboxyalcoxy en C1-C6; un groupement pipéridinosulfonyle ; un groupement pyrrolidino; un groupement alkyle(C1-C6)halogénoalkyle(C1-C6)amino; un groupement benzoylalkyle(C1-C6); un groupement vinyle ; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène;
   et M est un cation monovalent ou divalent issu de la famille des alcalins ou des alcalino-terreux, comme par exemple les ions sodium, potassium et calcium.
• Les dérivés STILBAZOLIUM de formule : 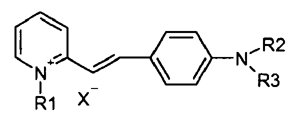 dans laquelle R1, R2 et R3 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo; un groupement amino; un groupement acylamino; un groupement dialkyl(C1-C6)amino ; un groupement dihydroxyalkyl(C1-C6)amino ; un groupement alkyl(C1-C6)hydroxyalkyl(C1-C6)amino; un groupement alcoxy(C1-C6); un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6); un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène;
   deux des substituants R2, R3 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4), et
   X- est un anion organique ou minéral. On peut citer par exemple pour X- les ions Chlorure, Bromure, Iodure, méthosulfate, éthosulfate, mésylate, tosylate, acétate, les sels d'acide organique simple tel que les lactate, les oléate, les benzoate, les perchlorate, les triflate.
• Les Dimères de Stilbazolium de formules: 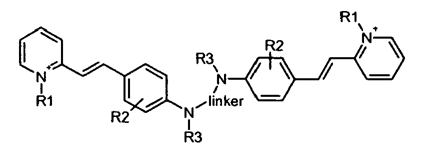 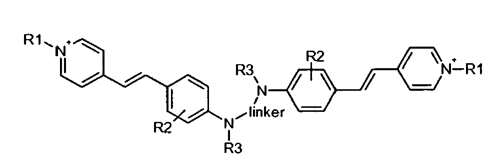 dans lesquelles R1, R2, R3 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo; un groupement amino; un groupement acylamino; un groupement dialkyl(C1-C6)amino ; un groupement dihydroxyalkyl(C1-C6)amino ; un groupement alkyl(C1-C6)hydroxyalkyl(C1-C6)amino; un groupement alcoxy(C1-C6); un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6); un groupement vinyle ; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
• Les Trimères et tétramères de Stilbazolium suivants 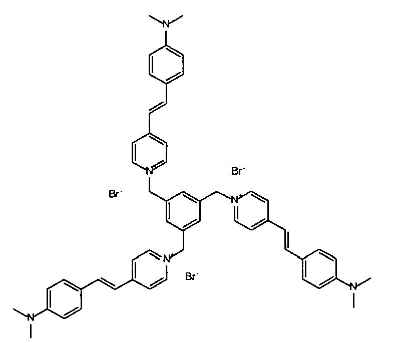 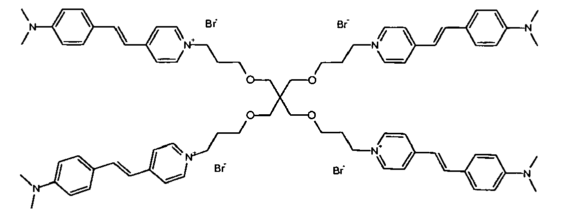
• Les dérivés Bis(BENZOXAZOLE) de formule : 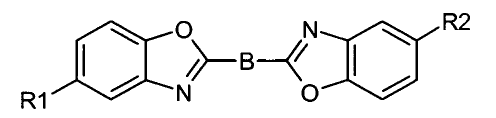 dans laquelle R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo; un groupement amino; un groupement acylamino; un groupement dialkyl(C1-C6)amino; un groupement dihydroxyalkyl(C1-C6)amino ; un groupement alkyl(C1-C6)hydroxyalkyl(C1-C6)amino; un groupement alcoxy(C1-C6); un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino; un groupement benzoylalkyle(C1-C6); un groupement vinyle ; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; et
   B est choisi parmi 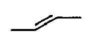 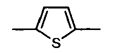 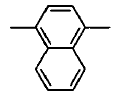 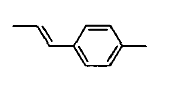
• Les Bis(BENZIMIDAZOLES) cationiques ou non
• Les 1,3-Diphenyl-2-PYRAZOLINES anioniques ou non, notamment 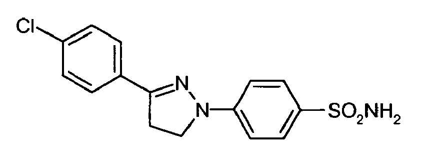
• Les DICETOPYRROLOPYRROLE de formule : 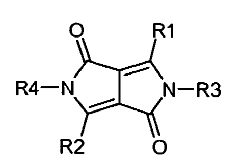 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino; un groupement acylamino ; un groupement dialkyl(C1-C6)amino ; un groupement dihydroxyalkyl(C1-C6)amino ; un groupement alkyl(C1-C6)hydroxyalkyl(C1-C6)amino; un groupement alcoxy(C1-C6); un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino; un groupement benzoylalkyle(C1-C6) ; un groupement vinyle ; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
   On peut citer par exemple le composé suivant 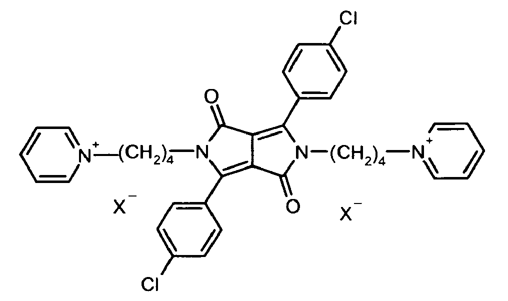 dans lequel X- est un anion défini comme précédemment.
• Les dérivés de DICYANO PYRAZINES de formules : 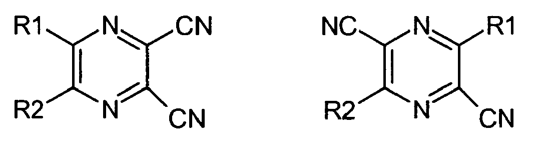 dans lesquelles R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo; un groupement amino; un groupement acylamino; un groupement dialkyl(C1-C6)amino ; un groupement dihydroxyalkyl(C1-C6)amino ; un groupement alkyl(C1-C6)hydroxyalkyl(C1-C6)amino; un groupement alcoxy(C1-C6); un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino; un groupement benzoylalkyle(C1-C6); un groupement vinyle ; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène;
   deux des substituants R1 et R2, peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non,insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4);

On peut citer les composés dans la publication suivante : « Selective topochemicalphotoreaction of crystallized 2,3-(-phenyletheny)-4,5-dicyanopyrazine" de Kim, Jae Hong; Matsuoka Masaru, Chem. Lett. (1999), (2), 143-144
On peut notamment citer

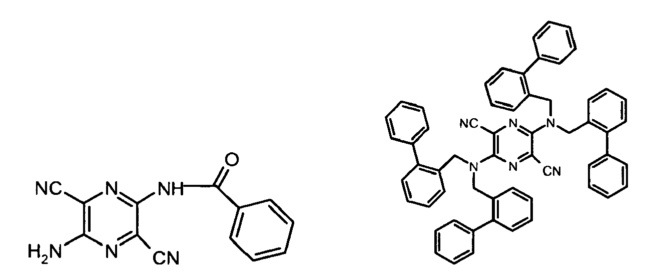

L'espèce colorée présente dans la composition de l'invention peut être un pigment.

Au sens de la présente invention, on entend par pigment toute entité organique et / ou minérale dont la solubilité dans l'eau est inférieure à 0,01 % à 20 °C, de préférence inférieure à 0,0001 %, et présentant une absorption entre 350 et 750 nm, de préférence une absorption avec un maximum.

Les pigments qui peuvent être utilisés sont notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : Ta2O5, Ti3O5, Ti2O3, TiO, ZrO2 en mélange avec TiO2, ZrO2, Nb2O5, CeO2, ZnS.

Le pigment non traité en surface, appelé par la suite pigment, peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

A titre d'exemple on peut aussi citer les pâtes pigmentaires de pigment organique telles que les produits vendus par la société HOECHST sous le nom:
- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane ou d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Prestige Bronze commercialisée par Eckart (Mica-Fe2O3),Colorona commercialisée par Merck (Mica-TiO2-Fe2O3).

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être synthétisées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré des pigments autres que les pigments blancs.

La taille du pigment utilisé dans la composition cosmétique selon la présente invention est généralement comprise entre 10 nm et 200 µm, de préférence entre 20 nm et 80 µm, et plus préférentiellement entre 30 nm et 50 µm.

Les pigments peuvent être dispersées dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C8 à C20 et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy12-stéarique sont de préférence destinés à un milieu hydrocarboné ou fluoré, alors que les mélanges de polydiméthylsiloxane oxyéthylène/oxypropyléné sont de préférence destinés à un milieu siliconé.

Les pigments utilisés dans la composition cosmétique selon l'invention peuvent être traités en surface par un agent organique.

Ainsi les pigments préalablement traités en surface utiles dans le cadre de l'invention sont des pigments ou des charges qui ont subi totalement ou partiellement un traitement de surface de nature chimique, électronique, électrochimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments.
Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :
- un traitement PEG-Silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL /SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

La composition conforme à la présente invention peut de plus comprendre un ou plusieurs pigments non traités en surface.

A titre d'espèces colorantes, on peut notamment citer les précurseurs de colorants tels que les bases d'oxydation et les coupleurs. Parmi les bases d'oxydation classiquement utilisées en teinture d'oxydation, on peut notamment citer les paraphénylènediamines, les bis-phénylalkylènediamines, les paraaminophénols, les ortho-aminophénols et les bases hétérocycliques. Parmi les coupleurs, on peut notamment citer les métaphénylènediamines, les méta-aminophénols, les métadiphénols, les naphtols et les coupleurs hétérocycliques tels que par exemple les dérivés indoliques, les dérivés indoliniques, les dérivés pyridiniques, les dérivés indazoliques, les dérivés de pyrazolo[1,5-b]-1,2,4-triazole, les dérivés de pyrazolo[3,2-c]-1,2,4-triazole, les dérivés de benzimidazole, les dérivés de benzothiazole, les dérivés de benzoxazole, les dérivés de 1,3-benzodioxole et les pyrazolones, et leurs sels d'addition avec un acide.

La concentration en espèce colorée ou colorante dans la composition est comprise entre 0.01 et 50%, de préférence entre 0.5 et 20%.

Selon un mode de réalisation particulier, l'espèce colorée est un pigment.

La composition utilisée selon l'invention peut également comprendre un ou plusieurs ingrédients cosmétiques supplémentaires.

Ce ou ces ingrédients sont généralement choisis parmi les silicones organomodifiées ou non autres que les polymères de l'invention telles que les silicones aminées, les polymères cationiques, anioniques, amphotères ou non ioniques fixants ou non, les peptides et leurs dérivés, les hydrolysats de protéines, les cires, les agents de gonflement et de pénétration tels que le diméthylisosorbitol, l'urée et ses dérivés, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les filtres solaires, les vitamines ou provitamines, les acides gras, les alcools gras, les esters gras, les huiles minérales, végétales ou synthétiques, ainsi que les parfums et les conservateurs, et leurs mélanges.

Généralement, le ou lesdits actifs cosmétiques supplémentaires représentent de 0,01 à 30%, de préférence de 0,1 à 10%, en poids du poids total de la composition cosmétique.

La composition selon l'invention peut comprendre un ou plusieurs solvants cosmétiquement acceptables choisis parmi l'eau, les alcools en C₁-C₆, de préférence les alcanols tels que l'éthanol, le propanol et l'isopropanol, les polyols tels que le propylène glycol, le glycérol et le pentanediol, l'alcool benzylique, les éthers de polyols, les esters en C₂-C₆, la N-méthylpyrrolidone (NMP), les cétones en C₃-C₆, et les huiles de silicone.

Si la composition est aqueuse son pH est généralement compris entre 2 et 13, de préférence entre 4 et 10.

Le réglage du pH de la composition peut être obtenu à l'aide d'un agent alcalin, tel que, par exemple, l'ammoniaque, la monoéthanolamine, la diéthanolamine, la triéthanolamine, la 1,3-propanediamine, un carbonate ou bicarbonate alcalin ou d'ammonium, un carbonate organique tel que le carbonate de guanidine, un hydroxyde alcalin, ou bien à l'aide d'un agent acidifiant tel que par exemple l'acide chlorhydrique, l'acide acétique, l'acide lactique, l'acide oxalique ou l'acide borique.

De préférence, la composition appliquée selon le procédé selon l'invention est anhydre, c'est-à-dire ne contient substantiellement pas d'eau et en tous cas moins de 1 % d'eau.

De préférence encore, le solvant utilisé est l'éthanol ou une huile de silicone.

La composition utilisée dans le procédé selon l'invention peut se présenter sous la forme d'une lotion, épaissie ou non, d'une crème ou d'un gel, d'une cire ou d'une mousse aérosol.

Les compositions peuvent être contenues dans un flacon pompe, un spray aérosol.

### ETAPES DE PROCEDE

De préférence, ladite composition est appliquée sur des fibres capillaires humides. Cette application peut être ou non suivie d'un rinçage.

Généralement, la composition appliquée sur les fibres capillaires est appliquée à hauteur de 0,05 à 0,3 g, de préférence de 0,1 à 0,2 g de composition par gramme de fibre capillaire sèche.

Après application de la composition, et avant l'élévation de la température des fibres capillaires on peut laisser poser ladite composition, généralement pendant 30 secondes à 60 minutes, de préférence 5 à 45 minutes.

Comme expliqué précédemment, le procédé selon l'invention comprend, après l'étape d'application de la composition, une étape d'élévation de la température des fibres capillaires à une température comprise entre 50 et 280°C. De préférence, la température des fibres capillaires est élevée jusqu'à une température comprise entre 60°C et 250°C, mieux entre 120°C et 220°C.

De préférence l'élévation de température est réalisée au moyen d'un fer.

Au sens de la présente invention, on entend par fer un dispositif de chauffage des fibres capillaires mettant en contact lesdites fibres et le dispositif de chauffage.

Les fers utilisables peuvent être des fers à boucler, des fers à lisser, des fers à cranter.

A titre d'exemple de fer utilisable dans le procédé selon l'invention, on peut citer tous types de fer plats ou ronds et, en particulier, de manière non limitative, ceux décrits dans les brevets US 4 103 145, US 4 308 878, US 5 983 903, US 5 957 140, US 5 494 058 et US 5 046 516.

Dans les fers plats l'extrémité du fer venant en contact avec les cheveux présente deux surfaces planes. Ces deux surfaces planes peuvent être métalliques. Elles peuvent être lisses ou crantées.

L'application du fer peut se faire par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage total ou partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque à une température inférieure à 50°C ou bien encore par séchage libre.

L'élévation de température peut être simultanée avec l'application de la composition selon l'invention.

De préférence l'élévation de température suit l'application de la composition selon l'invention.

Les modes de réalisation ci-dessus peuvent être mis en oeuvre en utilisant un dispositif comprenant une source de chaleur combinée à un réservoir de composition cosmétique contenant le polymère. A titre d'exemple un tel dispositif de conditionnement et d'application peut comprendre :
- Un réservoir, chauffé ou non chauffé,
- Une composition contenant un ou plusieurs polymères selon l'invention
- Un dispositif d'application,
- Un moyen de chauffage disposé de part et d'autre du dispositif d'application qui permet de chauffer simultanément ou postérieurement à son application la composition cosmétique à la température souhaitée. Le moyen de chauffage peut ainsi faire office d'applicateur.

Selon un mode de réalisation de l'invention, le traitement selon l'invention est combiné avec un traitement de la fibre existant

Ainsi dans une première étape on peut appliquer une permanente ou une coloration d'oxydation ou une décoloration ou un shampooing ou un produit de coiffage ou un défrisage alcalin et dans une seconde étape on procède au procédé décrits de l'invention. On renforce ainsi la durabilité du premier traitement.

Un autre procédé particulière intéressant consiste à appliquer le procédé selon l'invention avant des traitements de la fibre existants, connus pour dégrader la fibre kératinique (permanente, coloration d'oxydation, décoloration, défrisage). De cette façon on protège aussi la fibre lors du traitement.

Le procédé de la présente invention permet notamment de conférer facilement une forme thermoréversible aux dites fibres et d'améliorer durablement leur brillance et leur toucher.

L'invention est illustrée par les exemples suivants.

### Exemples :

On met en oeuvre le procédé de traitement des fibres capillaires selon l'invention les compositions suivantes. Les pourcentages sont donnés en poids par rapport au poids total de la composition.

### Exemple 1 :

| | |
|---|---|
| **POLYUREADIMETHICONE (WACKER-BELSIL UD 80)** | 2% |
| **CYCLOPENTASILOXANE** | 43% |
| **DOW CORNING 245 FLUID** | |
| **ETHANOL** | 55% |

1g de cette composition est appliqué sur une mèche de cheveux châtains de 2,7g préalablement shampooinée. La mèche est séchée au sèche-cheveux pendant 30min. La mèche est entourée autour d'un fer à friser à 200°C pour obtenir une « anglaise ».

Après traitement, les cheveux sont doux et brillants. La même mèche est ensuite lissée avec un fer plat puis rebouclée avec le fer à friser. La boucle obtenue est identique à celle de la première étape(thermoréversibilité).

### Exemple 2 :

| | |
|---|---|
| **POLYUREADIMETHICONE (WACKER-BELSIL** UD 80) | 2% |
| **CYCLOHEXASILOXANE DOW CORNING 246 FLUID** | 41% |
| **ETHANOL** | 55% |
| **Nacre Candurin Blue Shimmer de Merck** | 2% |

1g de cette composition est appliqué sur une mèche de cheveux châtains de 2,7g préalablement shampooinée. La mèche est séchée au sèche-cheveux pendant 30min. La mèche est entourée autour d'un fer à friser à 200°C pour obtenir une « anglaise » avec des reflets bleutés.La mèche est brillante et douce.

### Exemple 3 :

| | |
|---|---|
| **POLYUREADIMETHICONE (WACKER-BELSIL UD 80)** | 10% |
| **CYCLOPENTASILOXANE DOW CORNING 245 FLUID** | 35% |
| **ETHANOL** | 55% |

1g de cette composition est appliqué sur une mèche de cheveux châtains de 2,7g préalablement shampooinée. La mèche est séchée au sèche-cheveux à une température inférieure à 50°C pendant 30min. La mèche est structurée avec un fer à cranter à 200°C, des crans sont obtenus sur toute la surface de la mèche. Un shampooing est ensuite effectué. Le dépôt est toujours présent sur les cheveux et permet de repasser le fer à cranter pour obtenir le même effet de crans sur les cheveux que lors de la première étape avec un effet durable.

### Exemple 4 :

| | |
|---|---|
| **POLYUREADIMETHICONE (WACKER-BELSIL UD 80)** | 5% |
| **ISODODECANE** | 40% |
| **ETHANOL** | 55% |

1g de cette composition est appliqué sur une mèche de cheveux châtains de 2,7g préalablement shampooinée. La mèche est séchée au sèche-cheveux à une température inférieure à 50°C pendant 30min. La mèche est structurée avec un fer à cranter à 200°C, des crans sont obtenus sur toute la surface de la mèche. Un shampooing est ensuite effectué. Le dépôt est toujours présent sur les cheveux et permet de repasser le fer à cranter pour obtenir le même effet de crans sur les cheveux que lors de la première étape (effet durable).

### Exemple 5 :

| | |
|---|---|
| **POLYUREADIMETHICONE (WACKER-BELSIL UD 80)** | 5% |
| **ISODODECANE** | 30% |
| **ETHANOL** | 55% |
| **Nacre Prestige Soft de Eckart** | 10% |

1g de cette composition est appliqué sur une mèche de cheveux châtains de 2,7g préalablement shampooinée. La mèche est séchée au sèche-cheveux à une température inférieure à 50°C pendant 30min. La mèche est structurée avec un fer à cranter à 200°C, des crans sont obtenus sur toute la surface de la mèche ainsi qu'un reflet de couleur bronze. Un shampooing est ensuite effectué. Le dépôt est toujours présent sur les cheveux et permet de repasser le fer à cranter pour obtenir le même effet de crans sur les cheveux que lors de la première étape, la couleur est également toujours présente (effet durable).

## Revendications

1. Procédé de traitement des fibres capillaires comprenant les étapes suivantes :
- application sur les fibres capillaires d'une composition cosmétique comprenant, dans un milieu cosmétiquement acceptable, un ou plusieurs copolymères bloc polysiloxane polyurée, puis ou simultanément
- élévation de la température des fibres capillaires à une température comprise entre 50 et 280°C.

2. Procédé selon la revendication 1 dans lequel le copolymère est non ionique.

3. Procédé selon l'une quelconque des revendications 1 ou 2 dans lequel le copolymère contient un ou plusieurs blocs polyuréthane.

4. Procédé selon l'une quelconque des revendications précédentes 1 à 3 dans laquelle le copolymère contient uniquement des blocs polysiloxane et des blocs polyurée.

5. Procédé selon l'une quelconque des revendications précédentes dans laquelle le bloc polysiloxane du copolymère contient une quantité en poids de polysiloxane supérieure à 5 %.

6. Procédé selon la revendication 5 dans lequel la quantité de polysiloxane dans le copolymère est majoritaire dans le copolymère, de préférence supérieure à 90 % en poids par rapport au poids total du copolymère.

7. Procédé selon l'une quelconque des revendications précédentes dans lequel le copolymère polysiloxane/polyurée correspond à la formule générale (I) : 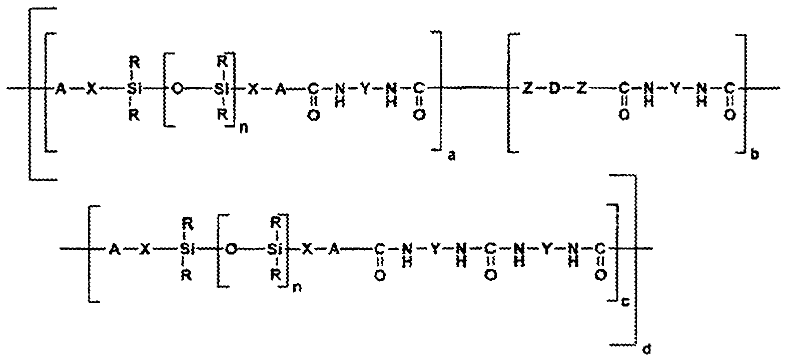 dans laquelle
R représente un radical hydrocarboné monovalent de 1 à 20 atomes de carbone, pouvant être substitué par un ou plusieurs atomes de fluor ou de chlore, ,
X représente un radical alkylène ayant 1 à 20 atomes de carbone, dans lequel des unités méthylène non voisines peuvent être remplacées par des radicaux -O-,
A représente un atome d'oxygène ou un radical amino -NR'-,
Z représente un atome d'oxygène ou un radical amino -NR'-,
R' représente hydrogène ou un radical alkyle ayant 1 à 10 atomes de carbone,
Y représente un radical hydrocarboné bivalent, le cas échéant substitué par le fluor ou le chlore, ayant 1 à 20 atomes de carbone,
D représente un radical alkylène, le cas échéant substitué par fluor, chlore, alkyle en C₁-C₆ ou ester alkylique en C₁-C₆, ayant 1 à 700 atomes de carbone, dans lequel des unités méthylène non voisines peuvent être remplacées par des radicaux -O-, -COO-, - OCO- ou -OCOO-,
n est un nombre allant de 1 à 4000,
a est un nombre d'au moins 1,
b est un nombre allant de 0 à 40,
c est un nombre allant de 0 à 30, et
d est un nombre supérieur à 0.
à la condition que A dans au moins un des motifs (a) représente un radical -NH-.

8. Procédé selon l'une quelconque des revendications précédentes dans lequel le copolymère est susceptible d'être obtenu par un procédé, qui comprend deux étapes telles que :
- dans la première étape, on fait réagir un silazane cyclique de la formule générale (2) ou (2') : 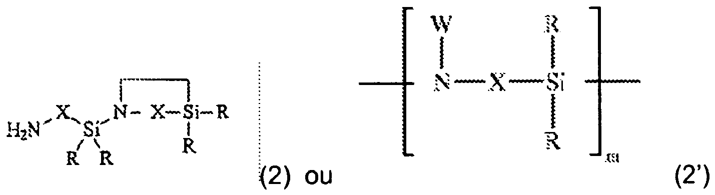 avec un composé organique du silicium de la formule générale (3) :
(HO)(R₂SiO)ₙ₋₁[H] (3)
en un aminoalkylpolydiorganosiloxane de la formule générale (4)
H₂N-X-[SiR₂O]ₙSiR₂-X-NH₂ (4)
- et dans une deuxième étape, l'aminoalkyl-polydiorganosiloxane de la formule générale (4) est polymérisé avec un diisocyanate de la formule générale (5) :
OCN-Y-NCO (5)
X, Y, R et n étant définis dans la revendication 1.

9. Procédé selon l'une quelconque des revendications précédentes dans lequel le copolymère répond au nom INCI polyureadimethicone.

10. Procédé selon l'une quelconque des revendications précédentes dans lequel le ou les copolymères bloc polysiloxane polyurée représentent de 0.05 à 20%, de préférence à 0.1 à 15% et encore plus préférentiellement de 0.5 à 10% du poids de la composition appliquée.

11. Procédé selon l'une quelconque des revendications précédentes **caractérisé en ce que** la composition comprend en outre un ou plusieurs ingrédients cosmétiques supplémentaires choisis parmi les silicones organomodifiés, les polymères cationiques, non ioniques, anioniques ou amphotères, les peptides et leurs dérivés, les hydrolysats de protéines, les cires, les agents de gonflement et de pénétration, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les filtres solaires, les vitamines ou provitamines, les acides gras, les alcools gras, les huiles minérales, végétales ou synthétiques, ainsi que les parfums et les conservateurs, et leurs mélanges.

12. Procédé selon l'une quelconque des revendications précédentes, **caractérisé en ce que** l'élévation de température suit l'application de la composition sur des fibres capillaires humides.

13. Procédé selon l'une quelconque des revendications précédentes **caractérisé en ce que** l'élévation de la température est comprise entre 60 et 250°C, et plus avantageusement entre 120 et 220°C.

14. Procédé selon l'une quelconque des revendications précédentes **caractérisé en ce qu'**il comprend en outre une étape de permanente ou de coloration d'oxydation ou de décoloration ou de défrisage.

## Patentansprüche

1. Verfahren zur Behandlung von Haarfasern, bei dem man:
- auf die Haarfasern eine kosmetische Zusammensetzung, die in einem kosmetisch unbedenkliches Medium ein oder mehrere Polysiloxan-Polyharnstoff-Blockcopolymere umfasst, ausbringt und danach oder gleichzeitig
- die Temperatur der Haarfasern auf eine Temperatur zwischen 50 und 280°C erhöht.

2. Verfahren nach Anspruch 1, bei dem das Copolymer nichtionisch ist.

3. Verfahren nach einem der Ansprüche 1 und 2, bei dem das Copolymer einen oder mehrere Polyurethanblöcke enthält.

4. Verfahren nach einem der vorhergehenden Ansprüche 1 bis 3, bei dem das copolymer nur Polysiloxanblöcke und Polyharnstoffblöcke enthält.

5. Verfahren nach einem der vorhergehenden Ansprüche, bei dem der Polysiloxanblock des Copolymers eine Polysiloxanmenge von mehr als 5 Gew.-% enthält.

6. Verfahren nach Anspruch 5, bei dem die Polysiloxanmenge in dem Copolymer in dem Copolymer in der Majorität und vorzugsweise mehr als 90 Gew.-%, bezogen auf das Gesamtgewicht des Copolymers, vorliegt.

7. Verfahren nach einem der vorhergehenden Ansprüche, bei dem das Polysiloxan/Polyharnstoff-Copolymer der allgemeinen Formel (I) entspricht: 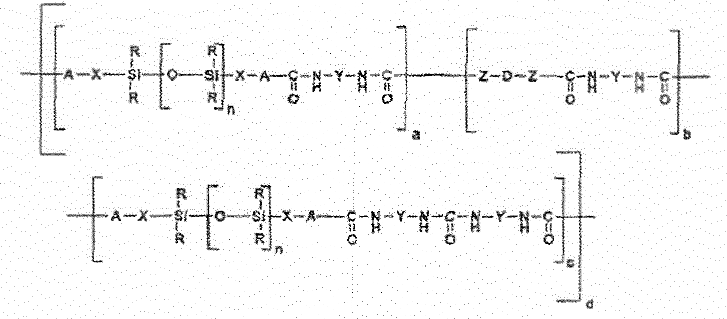 worin
R für einen einwertigen Kohlenwasserstoffrest mit 1 bis 20 Kohlenstoffatomen, der durch ein oder mehrere Fluor- oder Chloratome substituiert sein kann, steht,
X für einen Alkylenrest mit 1 bis 20 Kohlenstoffatomen, in dem nicht benachbarte Methyleneinheiten durch-O-Reste ersetzt sein können, steht,
A für ein Sauerstoffatom oder einen Aminorest -NR'- steht,
Z für ein Sauerstoffatom oder einen Aminorest -NR'- steht,
R' für Wasserstoff oder einen Alkylrest mit 1 bis 10 Kohlenstoffatomen steht,
Y für einen zweiwertigen Kohlenwasserstoffrest, der gegebenenfalls durch Fluor oder Chlor substituiert ist und 1 bis 20 Kohlenstoffatome aufweist, steht,
D für einen Alkylenrest, der gegebenenfalls durch Fluor, Chlor, C₁₋₆-Alkyl oder C₁₋₆-Alkylester substituiert ist und 1 bis 700 Kohlenstoffatome aufweist, in dem nicht benachbarte Ethyleneinheiten durch -O-, -COO-, -OCO- oder -OCOO-Reste ersetzt sein können, steht,
n für eine Zahl im Bereich von 1 bis 4000 steht,
a für eine Zahl von mindestens 1 steht,
b für eine Zahl im Bereich von 0 bis 40 steht,
c für eine Zahl im Bereich von 0 bis 30 steht und
d für eine Zahl größer 0 steht,
mit der Maßgabe, dass A in mindestens einer der (a) -Einheiten für einen -NH-Rest steht.

8. Verfahren nach einem der vorhergehenden Ansprüche, bei dem das Copolymer nach einem zwei schritte umfassenden Verfahren erhältlich ist, bei dem man:
- in einem ersten Schritt ein cyclisches Silazan der allgemeinen Formel (2) oder (2'): 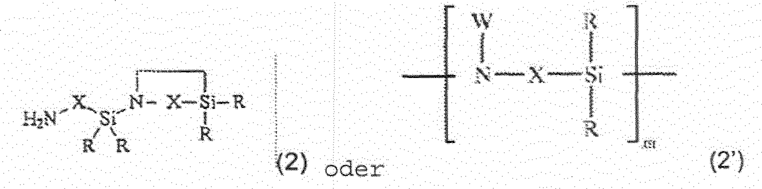 mit einer organischen Siliciumverbindung der allgemeinen Formel (3):
(HO) (R₂SiO)ₙ₋₁[H] (3)
zu einem Aminoalkylpolydiorganosiloxan der allgemeinen Formel (4):
H₂N-X- [SiR₂O]ₙSiR₂-X-NH₂ (4)
umsetzt
- und in einem zweiten Schritt das Aminoalkylpolydiorganosiloxan der allgemeinen Formel (4) mit einem Diisocyanat der allgemeinen Formel (5):
OCN-Y-NCO (5)
polymerisiert,
wobei X, Y, R und n in Anspruch 1 definiert sind.

9. Verfahren nach einem der vorhergehenden Ansprüche, bei dem das copolymer dem INCI-Namen Polyureadimethicone entspricht.

10. Verfahren nach einem der vorhergehenden Ansprüche, bei dem das oder die Polysiloxan-Polyharnstoff-Blockcopolymere 0,05 bis 20 Gew.-%, vorzugsweise 0,1 bis 15 Gew.-% und noch weiter bevorzugt 0,5 bis 10 Gew.-% der aufgebrachten Zusammensetzung ausmachen.

11. Verfahren nach einem der vorhergehenden Ansprüche, **dadurch gekennzeichnet, dass** die Zusammensetzung außerdem auch noch einen oder mehrere zusätzliche kosmetische Bestandteils enthält, die aus organisch modifizierten Silikonen, kationischen, nichtionischen, anionischen oder amphoteren Polymeren, Peptiden und Derivaten davon, Proteinhydrolysaten, Wachsen, Quellmittel, Penetriermitteln, anionischen, kationischen, nichtionischen, amphoteren oder zwitterionischen Tensiden, Mitteln zur Bekämpfung von Haarverlust, Antischuppenmitteln, natürlichen oder synthetischen assoziativen oder nichtassoziativen Verdickern, Suspendiermitteln, Sequestriermitteln, Lichtschutzmitteln, Vitaminen oder Provitaminen, Fettsäuren, Fettalkoholen, mineralischen, pflanzlichem oder synthetischen Ölen sowie Duftstoffen und Konservierungsmitteln und Mischungen davon ausgewählt sind.

12. Verfahren nach einem des vorhergehenden Ansprüche, **dadurch gekennzeichnet, dass** die Erhöhung der Temperatur auf das Aufbringen der Zusammensetzung auf die feuchten Haarfasern folgt.

13. Verfahren nach einem der vorhergehenden Ansprüche, **dadurch gekennzeichnet, dass** die Erhöhung der Temperatur auf eine Temperatur zwischen 60 und 250°C und vorteilhafter zwischen 120 und 220°C erfolgt.

14. Verfahren nach einem der vorhergehenden Ansprüche, **dadurch gekennzeichnet, dass** es außerdem einen Schritt der Dauerwellung oder Oxidationsfärbung oder Bleichung oder Entkräuselung umfasst.

## Claims

1. Process for treating hair fibres that comprises the following steps:
- applying a cosmetic composition comprising, in a cosmetically acceptable medium, one or more polysiloxane/polyurea block copolymers to the hair fibres; then or simultaneously
- raising the temperature of the hair fibres to a temperature between 50 and 280°C.

2. Process according to Claim 1, in which the copolymer is non-ionic.

3. Process according to either one of Claims 1 and 2, in which the copolymer contains one or more polyurethane blocks.

4. Process according to any one of the preceding claims 1 to 3, in which the copolymer contains only polysiloxane blocks and polyurea blocks.

5. Process according to any one of the preceding claims, in which the polysiloxane block of the copolymer contains an amount, by weight, of polysiloxane greater than 5%.

6. Process according to Claim 5, in which the amount of polysiloxane in the copolymer is in the majority in the copolymer, preferably greater than 90% by weight relative to the total weight of the copolymer.

7. Process according to any one of the preceding claims, in which the polysiloxane/polyurea copolymer corresponds to the general formula (I): 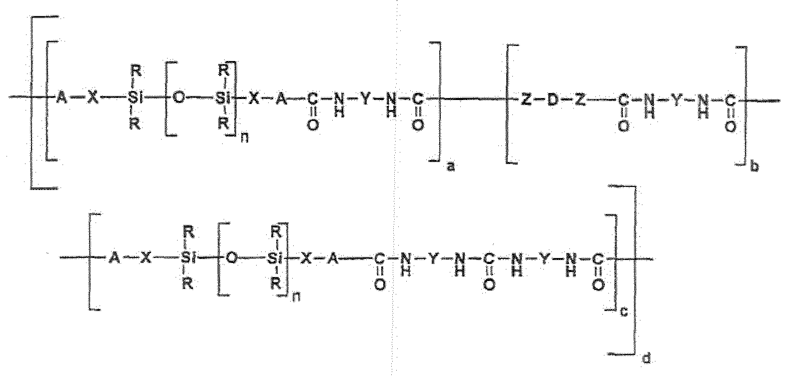 in which:
R represents a monovalent hydrocarbon-based radical having 1 to 20 carbon atoms, which may be substituted by one or more fluorite or chlorine atoms;
X represents an alkylene radical having 1 to 20 carboy atoms, in which non-neighbouring methylene units may be replaced by -O- radicals;
A represents an oxygen atom or an -NR'- amino radical;
Z represents an oxygen atom or an -NR'- amino radical;
R' represents hydrogen or an alkyl radical having 1 to 10 carbon atoms;
Y represents a bivalent hydrocarbon-based radical, where appropriate substituted by fluorine or chlorine, having 1 to 20 carbon atoms;
D represents an alkylene radical, where appropriate substituted by fluorine, chlorine, C₁-C₆ alkyl or C₁-C₆ alkyl ester, having 1 to 700 carbon atoms, in which non-neighbouring methylene units may be replaced by -O-, -COO-, -OCO- or -OCOO- radicals;
n is a number ranging from 1 to 4000;
a is a number of at least 1;
b is a number ranging from 0 to 40;
c is a number ranging from 0 to 30; and
d is a number greater than 0,
on condition that A in at least one of the (a) units represents an -NH- radical.

8. Process according to any one of the preceding claims, in which the copolymer is capable of being obtained by a process that comprises two steps such that:
- in the first step, a cyclic silazane of general formula (2) or (2'): 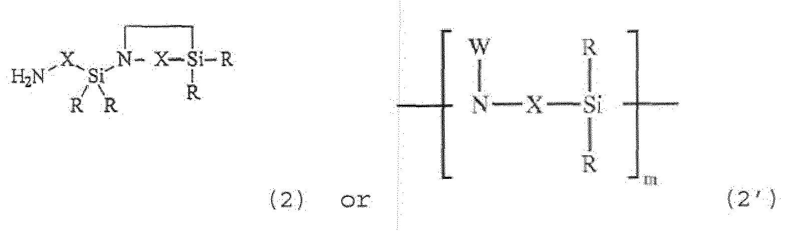 is reacted with an organic silicon compound of general formula (3):
(HO) (R₂SiO)ₙ₋₁[H] (3)
to give an aminoalkylpolydiorganosiloxane of general formula (4):
H₂N-X- [SiR₂O]ₙSiR₂-X-NH₂ (4);
- and in a second step, the aminoalkylpolydiorganosiloxane of general formula (4) is polymerized with a diisocyanate of general formula (5):
OCN-Y-NCO (5),
X, Y, R and n being defined in Claim 1.

9. Process according to any one of the preceding claims, in which the copolymer corresponds to the INCI name: polyureadimethicone.

10. Process according to any one of the preceding claims, in which the polysiloxane/polyurea block copolyme(s) represent(s) from 0.05 to 20%, preferably from 0.1 to 15% and more preferably still from 0.5 to 10% of the weight of the composition applied.

11. Process according to any one of the preceding claims, **characterized in that** the composition also comprises one or more supplementary cosmetic ingredients chosen from organomodified silicones, cationic, non-ionic, anionic or amphoteric polymers, peptides and derivatives thereof, protein hydrolysates, waxes, swelling agents, penetrants, anionic, cationic, non-ionic, amphoteric or zwitterionic surfactants, agents for combatting hair loss, anti-dandruff agents, natural or synthetic associative or non-associative thickeners, suspension agents, sequestering agents, sunscreens, vitamins or provitamins, fatty acids, fatty alcohols, mineral, plant or synthetic oils, and also fragrances and preservatives and mixtures thereof.

12. Process according to any one of the preceding claims, **characterized in that** the temperature rise follows the application of the composition to wet hair fibres.

13. Process according to any one of the preceding claims, **characterized in that** the temperature rise is between 60 and 250°C, and more advantageously between 120 and 220°C.

14. Process according to any one of the preceding claims, **characterized in that** it also comprises a step of permanent waving or oxidation dyeing or bleaching or straightening.
